# EUROPEAN PATENT APPLICATION

(11) **EP 4 578 847 A1**
(43) Date of publication of application: **02.07.2025**
(21) Application number: 24197535.8
(22) Date of filing: 30.08.2024
(51) Int. Cl.: C07C 51/41, C07C 55/07

(54) **A METHOD FOR PREPARING AN IN SITU METAL DOPED MANGANESE IRON OXALATE PRECURSOR**

(30) Priority: 28.12.2023 CN 202311827527
(71) Applicant: Hunan Huaxing Lithium Electric New Energy Co. , Ltd., Changsha City, Hunan Province 410600 (CN)
(72) Inventor: TANG, Yuanlin, Shaoyang, 422900 (CN); CHEN, Hailin, Liling, 412212 (CN); FU, Zhao, Guangzhou, 510000 (CN); XU, Changmei, Huaihua City, 419600 (CN)
(74) Representative: Valet Patent Services Limited

(57) **Abstract**

A method for preparing an in situ metal doped manganese iron oxalate precursor is disclosed. Weighing iron source, manganese source, doped metal elements (Mg, Zn, etc.) according to a stoichiometric ratio, adding deionized water to ultrasonically dissolve to obtain a mixed metal solution. The mixed metal solution is added to a mixed solution of a precipitant and a complexant, and then a co-precipitation reaction is occurred under the protection of inert gas. A suspension of metal doped manganese iron oxalate is generated and then filtered, washed and dried to obtain a powder of manganese iron oxalate precursor. The method can effectively avoid the problem of uneven mixing due to the iron and manganese sources. Moreover, lithium manganese iron phosphate synthesized from the metal-doped manganese iron oxalate precursor can largely improve the electronic conductivity and also the electrochemical performance of lithium manganese iron phosphate.

## Description

### TECHNICAL FIELD

The present application relates to the technical field of inorganic fine chemicals, and specifically to a method for preparing an in situ metal-doped manganese iron oxalate precursor.

### BACKGROUND

Lithium-ion batteries, by virtue of their unique advantages such as high operating voltage, no memory effect, high discharge specific capacity, large safety factor, etc., and meanwhile by virtue of their good cycling stability, light weight, small size, etc., show extensive utilization value and potential economic benefits in various aspects such as portable electronic equipment, industrial production, electric vehicle market, etc. Therefore, as a new type of green and non-polluting energy source, the lithium-ion batteries have been attracted more and more general attention in recent years.

Most of iron and manganese sources used in the existing lithium-ion batteries are easy to be mixed unevenly, and the synthesized materials do not have high electronic conductivity and poor electrochemical performance, which cannot meet the use requirements of the lithium-ion batteries. In this regard, the present disclosure proposes a method for preparing an in situ metal-doped manganese iron oxalate precursor.

### SUMMARY

The present disclosure aims to provide a method for preparing an in situ metal-doped manganese iron oxalate precursor, to address problems that most of the iron and manganese sources used in the existing lithium-ion batteries are easy to be mixed unevenly, and that the synthesized materials do not have high electronic conductivity and poor electrochemical performance, which cannot meet the use requirements of the lithium-ion batteries.

To achieve above objectives, the present disclosure adopts the following technical solutions.

In a first aspect, in some embodiments of the present disclosure, a method for preparing an in situ metal-doped manganese iron oxalate precursor is provided, including the following steps of S1 to S6.

S1, weighing a ferric salt, a solid of manganese salt and other doped metal salts according to a chemical molar ratio, ultrasonic dissolving by adding appropriate amount of deionized water, to obtain a mixed metal solution with a concentration of 0.3~3.0 mol/L.

S2, weighing a precipitant and a complexant according to a molar ratio of the mixed metal solution to the precipitant of 1:1-1.5, ultrasonic dissolving by adding the deionized water, to obtain a mixed solution, wherein a concentration of a precipitant solution is 0.3~3.0 mol/L, and a concentration of the complexant is 0.1~1.0 mol/L.

S3, adding the mixed solution into a reaction kettle, sealing the reaction kettle and bubbling for deoxygenation, and then heating and stirring.

S4, after stabilization of a heating temperature, adding the mixed metal solution dropwise into the mixed solution in the reaction kettle at a certain flow rate.

S5, obtaining a suspension of manganese iron oxalate by reacting 0.5~48 h at a constant temperature under protection of an inert gas.

S6, after reaction finished, taking the suspension of manganese iron oxalate out of the reaction kettle, filtering, washing and drying, to obtain a powder of the manganese iron oxalate precursor.

Preferably, the ferric salt in S1 includes, but is not limited to, one or more of ferrous nitrate, ferrous sulfate, ferrous acetate, and ferrous chloride; and the ferrous sulfate is the ferrous sulfate without crystalline water or with crystalline water.

Preferably, the manganese salt in S1 includes, but is not limited to, one or more of manganous nitrate, manganous sulfate, manganous acetate, and manganous chloride, and the manganous sulfate is manganous sulfate without crystalline water or with crystalline water.

Preferably, other doped metal elements of the other doped metal salts in S1 includes, but is not limited to, one or more of Mg and Zn, and further includes one or more of Co, Ni, Al, Cu, Zr, Cr, and V, and the other doped metal salts is an amorphous metal salt or a crystalline metal salt.

Preferably, the chemical molar ratio of the ferric salt to the manganese salt is 1:1-5, and the concentration of the mixed metal solution is 0.3~3.0 mol/L. And a molar ratio of the other doped metal elements to manganese element in S1 is 1:6-120, and a molar ratio of the other doped metal elements to iron element is 1:4~80.

Preferably, the precipitant in S2 is an oxalate, and the oxalate includes, but is not limited to, one or more of oxalic acid, potassium oxalate, and sodium oxalate.

Preferably, a molar ratio of the mixed metal solution to the precipitant in S2 is 1:1-1.5, the concentration of the precipitant solution is 0.3~3.0 mol/L, and a bubbling gas used in S3 is one of argon, nitrogen and helium.

Preferably, the complexant is an oxalate, and the oxalate comprises at least one or more of ammonium oxalate, ammonium sulfate and ammonium carbonate. More preferably, the ammonium oxalate is ammonium oxalate monohydrate.

Preferably, in S4, the heating temperature is 20-100 °C, and a dropwise flow rate of the precipitant is 0.01~1.0 L/min.

Preferably, the inert gas in S5 is one of argon, nitrogen, helium, with a reaction temperature is 20-100 °C and a reaction time is 0.5~48 h.

In a second aspect, in some embodiments of the present disclosure, an in situ metal-doped manganese iron oxalate precursor is provided, prepared by the method described in the above first aspect of the present disclosure.

The method for preparing the in situ metal-doped manganese iron oxalate precursor of the present disclosure has at least the following advantages and beneficial effects.

(1) The method for preparing the in situ metal-doped manganese iron oxalate precursor of the present disclosure, the manganese iron oxalate precursor prepared by using a co-precipitation method can be mixed with lithium source to synthesize a cathode material of lithium manganese iron phosphate through a solid phase method. Compared with other iron and manganese sources, by using the manganese iron oxalate precursor as the iron and manganese sources, this can effectively avoid the problem of uneven mixing due to the iron and manganese sources. Moreover, lithium manganese iron phosphate synthesized from the metal-doped manganese iron oxalate precursor can largely improve the electronic conductivity and also the electrochemical performance of lithium manganese iron phosphate.

(2) The method for preparing the in situ metal-doped manganese iron oxalate precursor of the present disclosure, oxalates are not easy to introduce impurity phases during synthesis of the cathode material, the crystallinity is higher and the bonding force is larger, which are helpful to stabilize a skeleton structure of the synthesized product. And during the reaction process, it will decompose and release gases, which can inhibit the agglomeration of particles and the growth of grains.

(3) The method for preparing the in situ metal-doped manganese iron oxalate precursor of the present disclosure, not only the obtained produce has high purity, uniform powder size distribution and stable thermodynamic properties, but also the process is simple and reasonable, easy to industrialize large-scale production, with good economic benefits, and thus it is a preferred choice of raw materials for preparing the cathode material for power batteries.

### BRIEF DESCRIPTION OF THE DRAWINGS

FIG. 1 shows an X-ray diffraction (XRD) image of Mn_{0.6}Fe_{0.38}Mg_{0.02}C₂O₄·2H₂O in accordance with Example 1 of the present disclosure.
FIG. 2 shows a scanning electron microscope (SEM) image of Mn_{0.6}Fe_{0.38}Mg_{0.02}C₂O₄·2H₂O in accordance with Example 1 of the present disclosure.

### DETAILED DESCRIPTION OF THE EMBODIMENTS

The technical solutions in the embodiments of the present disclosure are described clearly and completely below, and it is obvious that the described embodiments are only a part of the embodiments of the present disclosure and not all of the embodiments. Based on the embodiments in the present disclosure, all other embodiments obtained by a person of ordinary skill in the art without creative labor shall fall within the scope of protection of the present invention.

The technical solutions of the present disclosure are described below.

In some embodiments of the present disclosure, a method for preparing an in situ metal doped manganese iron oxalate precursor is provided, including the following steps of S1 to S6.

S1, weighing a ferric salt, a solid of manganese salt and other doped metal salts according to a chemical molar ratio, ultrasonic dissolving by adding appropriate amount of deionized water, to obtain a mixed metal solution with a concentration of 0.3~3.0 mol/L.

S2, weighing a precipitant and a complexant according to a molar ratio of the mixed metal solution to the precipitant of 1:1-1.5, ultrasonic dissolving by adding the deionized water, to obtain a mixed solution, where a concentration of a precipitant solution is 0.3~3.0 mol/L, and a concentration of the complexant is 0.1~1.0 mol/L.

S3, adding the mixed solution into a reaction kettle, sealing the reaction kettle and bubbling for deoxygenation, and then heating and stirring.

S4, after stabilization of a heating temperature, adding the mixed metal solution dropwise into the mixed solution in the reaction kettle at a certain flow rate.

S5, obtaining a suspension of manganese iron oxalate by reacting 0.5~48 h at a constant temperature under protection of an inert gas.

S6, after reaction finished, taking the suspension of manganese iron oxalate out of the reaction kettle, filtering, washing and drying, to obtain a powder of the manganese iron oxalate precursor.

Herein the ferric salt in S1 includes, but is not limited to, one or more of ferrous nitrate, ferrous sulfate, ferrous acetate, and ferrous chloride; and the ferrous sulfate is the ferrous sulfate without crystalline water or with crystalline water. Specifically the ferrous sulfate may be one or more of anhydrous ferrous sulfate, ferrous sulfate monohydrate, and ferrous sulfate heptahydrate.

Herein the manganese salt in S1 includes, but is not limited to, one or more of manganous nitrate, manganous sulfate, manganous acetate, and manganous chloride; and the manganous sulfate is manganous sulfate without crystalline water or with crystalline water. Specifically the manganous sulfate may be one or more of anhydrous manganous sulfate, manganese sulfate monohydrate, and manganese sulfate tetrahydrate.

Herein other doped metal elements of the other doped metal salts in S1 include, but is not limited to, one or more of Mg and Zn, and further include one or more of Co, Ni, Al, Cu, Zr, Cr, and V, and the other doped metal salts may be an amorphous metal salt or a crystalline metal salt. Mg salt may specifically be one or more of anhydrous magnesium sulfate, magnesium acetate, and magnesium sulfate heptahydrate. Zn salt may specifically be one or more of anhydrous zinc sulfate, zinc acetate, and zinc sulfate heptahydrate.

Herein the chemical molar ratio of the ferric salt to the manganese salt is 1:1-5, and the concentration of the mixed metal solution is 0.3~3.0 mol/L.

Herein a molar ratio of the other doped metal elements to manganese element in S1 is 1:6-120, and a molar ratio of the other doped metal elements to iron element is 1:4~80.

Herein the precipitant in S2 is an oxalate, and the oxalate include, but is not limited to, one or more of oxalic acid, potassium oxalate, and sodium oxalate.

Herein the oxalate is one or more of oxalic acid, potassium oxalate, and sodium oxalate.

Herein a molar ratio of the mixed metal solution to the precipitant in S2 is 1:1-1.5, the concentration of the precipitant solution is 0.3- 3.0 mol/L.

Herein a bubbling gas used in S3 is one of argon, nitrogen and helium.

Herein in S4, the heating temperature is 20-100 °C, and a dropwise flow rate of the precipitant is 0.01~1.0 L/min.

Herein the inert gas in S5 is one of argon, nitrogen, helium, with a reaction temperature is 20-100 °C and a reaction time is 0.5~48 h.

In the present disclosure, by preparing the mixed metal solution, oxalic acid solution (i.e., the precipitant solution) and the complexant, a co-precipitation reaction under inert gas protection is occurred, and then filtering, washing, and drying, the in situ metal doped manganese iron oxalate precursor with thermodynamically stable is thus obtained. And in situ doping of other metal elements (Mg, Zn, etc.) in manganese iron oxalate precursor can be used for the synthesis of the anode material of lithium manganese iron phosphate, which can overcome the problems of poor intrinsic electronic conductivity of lithium manganese iron phosphate, and also inhomogeneous mixing of metal elements, thereby improving the electrochemical performance.

Based on the above methods, the present disclosure will provides the following partial specific examples.

The raw materials, reagents, or devices used in the following examples are, if not otherwise specified, commercially available from conventional sources, or can be obtained by existing known methods.

### Example 1

A method for preparing an in situ metal doped manganese iron oxalate precursor, with a chemical formula of Mn_{0.6}Fe_{0.38}Mg_{0.02}C₂O₄·2H₂O, including the following steps 1 to 6.

Step 1, according to a molar ratio of 4:6, 422.58 g of ferrous sulfate heptahydrate, 405.65 g of manganese sulfate monohydrate, and 19.18 g of magnesium sulfate heptahydrate are weighted in a beaker, 4L of deionized water is added and ultrasonically stirred until all metal salts are completely dissolved, a mixed metal solution with a concentration of 1.0 mol/L is obtained.

Step 2, 544.62 g of oxalic acid dihydrate and 56.84 g of ammonium oxalate monohydrate (i.e., the complexant) are weighted in the beaker, 4L of deionized water is added and ultrasonically stirred until they are all completely dissolved, to obtain a mixed solution, in which oxalic acid solution with a concentration of 1.08 mol/L and a complexant solution with a concentration of 0.1 mol/L.

Step 3, the above mixed solution is poured into a reaction kettle, the stirring is turned on at 400 r/min, and under the protection of nitrogen, oxygen is removed by continuous bubbling for 10 min, a reaction temperature is set at 40 °C.

Step 4, when the reaction temperature is stabilized at 40 °C, the mixed metal solution is slowly added dropwise at a flow rate of 0.08 L/min to the reaction kettle containing the mixed solution, and a yellow precipitate of manganese iron oxalate is immediately generated in the reaction kettle.

Step 5, after all of the mixed metal solution is added dropwise to the reaction kettle containing the mixed solution, the reaction is continued at 40 °C for 8 h under the protection of nitrogen, to obtain a suspension of manganese iron oxalate.

Step 6, after the reaction, the suspension of manganese iron oxalate is taken out from the reaction kettle, filtered under reduced pressure and washed repeatedly with deionized water until the filtrate is clarified, and the filter cake is taken and dried under vacuum at 80 °C for 12 h, to obtain the powder of manganese iron oxalate precursor.

### Example 2

A method for preparing an in situ metal doped manganese iron oxalate precursor, with a chemical formula of Mn_{0.6}Fe_{0.38}Zn_{0.02}C₂O₄·2H₂O, including the following steps 1 to 6.

Step 1, according to a molar ratio of 4:6, 422.58 g of ferrous sulfate heptahydrate, 405.65 g of manganese sulfate monohydrate, and 23 g of Zinc sulfate heptahydrate are weighted in a beaker, 4L of deionized water is added and ultrasonically stirred until all metal salts are completely dissolved, a mixed metal solution with a concentration of 1.0 mol/L is obtained.

Step 2, 544.62 g of oxalic acid dihydrate and 56.84 g of ammonium oxalate monohydrate (i.e., the complexant) are weighted in the beaker, 4L of deionized water is added and ultrasonically stirred until they are all completely dissolved, to obtain a mixed solution, in which oxalic acid solution with a concentration of 1.08 mol/L and a complexant solution with a concentration of 0.1 mol/L.

Step 3, the above mixed solution is poured into a reaction kettle, the stirring is turned on at 400 r/min, and under the protection of nitrogen, oxygen is removed by continuous bubbling for 10 min, a reaction temperature is set at 40 °C.

Step 4, when the reaction temperature is stabilized at 40 °C, the mixed metal solution is slowly added dropwise at a flow rate of 0.08 L/min to the reaction kettle containing the mixed solution, and a yellow precipitate of manganese iron oxalate is immediately generated in the reaction kettle.

Step 5, after all of the mixed metal solution is added dropwise to the reaction kettle containing the mixed solution, the reaction is continued at 40 °C for 8 h under the protection of nitrogen, to obtain a suspension of manganese iron oxalate.

Step 6, after the reaction, the suspension of manganese iron oxalate is taken out from the reaction kettle, filtered under reduced pressure and washed repeatedly with deionized water until the filtrate is clarified, and the filter cake is taken and dried under vacuum at 80 °C for 12 h, to obtain the powder of manganese iron oxalate precursor.

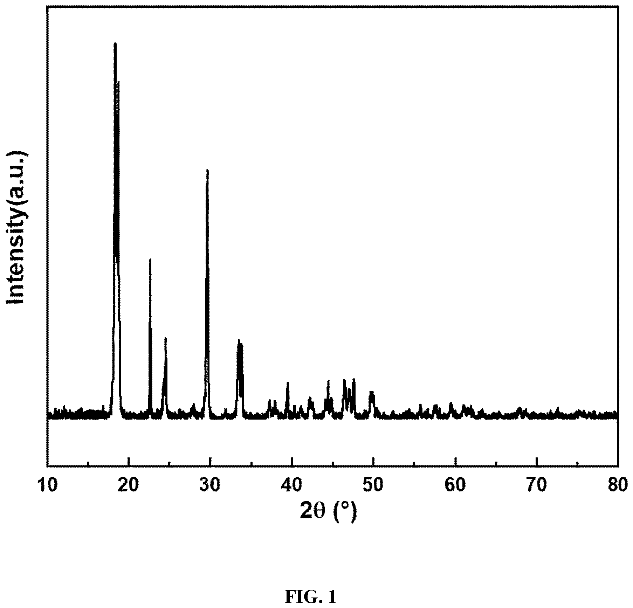

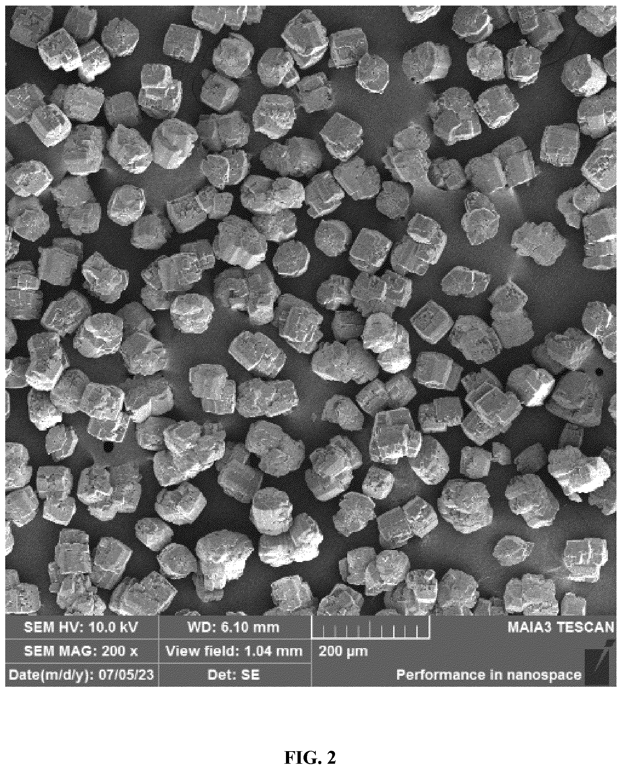

The product obtained from the Example 1 is subjected to XRD and SEM testing, and the results are shown in FIG. 1 and FIG. 2.

The method of the present disclosure for synthesizing lithium manganese iron phosphate by using manganese iron oxalate precursor as the raw material has the following advantages. Firstly, the oxalate is not easy to introduce impurity phases during synthesis of the anode material. Secondly, the anode material of lithium manganese iron phosphate synthesized by manganese iron oxalate precursor has a higher crystallinity and a larger bonding force, which can help to stabilize the skeleton structure of the synthesized product. And last, manganese iron oxalate precursor decomposes and releases gases during the reaction process, which can inhibit the agglomeration of particles and the growth of grains.

The above shows and describes the basic principles of the present disclosure, the main features and the advantages of the present disclosure, and it is apparent to one of ordinary skill in the art that, the present disclosure is not limited to the details of the above exemplary embodiments, and that it is capable of being realized in other specific forms without departing from the concept or the basic features of the present invention. Accordingly, the embodiments are to be regarded as exemplary and non-limiting in any point of view, and the scope of the present invention is limited by the appended claims and not by the foregoing description, and is therefore intended to encompass all variations falling within the meaning and scope of the equivalent elements of the claims.

Although embodiments of the present disclosure have been shown and described, it will be understood to one of ordinary skill in the art that a variety of changes, modifications, substitutions and variations may be made to these embodiments without departing from the principle and concept of the present invention, the scope of which is limited by the appended claims and their equivalents.

## Claims

1. A method for preparing an in situ metal doped manganese iron oxalate precursor, comprising steps of,
| | |
|---|---|
| S1, | weighing a ferric salt, a solid of manganese salt and other doped metal salts according to a chemical molar ratio, ultrasonic dissolving by adding appropriate amount of deionized water, to obtain a mixed metal solution with a concentration of 0.3~3.0 mol/L; |
| S2, | weighing a precipitant and a complexant according to a molar ratio of the mixed metal solution to the precipitant of 1:1∼1.5, ultrasonic dissolving by adding the deionized water, to obtain a mixed solution; wherein a concentration of a precipitant solution is 0.3~3.0 mol/L, and a concentration of the complexant is 0.1~1.0 mol/L; |
| S3, | adding the mixed solution into a reaction kettle, sealing the reaction kettle and bubbling for deoxygenation, and then heating and stirring; |
| S4, | after stabilization of a heating temperature, adding the mixed metal solution dropwise into the mixed solution in the reaction kettle at a certain flow rate; |
| S5, | obtaining a suspension of manganese iron oxalate by reacting 0.5~48 h at a constant temperature under protection of an inert gas; and |
| S6, | after reaction finished, taking the suspension of manganese iron oxalate out of the reaction kettle, filtering, washing and drying, to obtain a powder of the manganese iron oxalate precursor. |

2. The method according to claim 1, wherein the ferric salt in S1 comprises, one or more of ferrous nitrate, ferrous sulfate, ferrous acetate, and ferrous chloride; and the ferrous sulfate is the ferrous sulfate without crystalline water or with crystalline water.

3. The method according to claim 1, wherein the manganese salt in S1 comprises one or more of manganous nitrate, manganous sulfate, manganous acetate, and manganous chloride, and the manganous sulfate is manganous sulfate without crystalline water or with crystalline water.

4. The method according to claim 1, wherein other doped metal elements of the other doped metal salts in S1 comprises one or more of Mg and Zn, and further comprise one or more of Co, Ni, Al, Cu, Zr, Cr, and V, and the other doped metal salts is an amorphous metal salt or a crystalline metal salt.

5. The method according to claim 1, wherein the chemical molar ratio of the ferric salt to the manganese salt is 1:1-5, and the concentration of the mixed metal solution is 0.3~3.0 mol/L; and wherein a molar ratio of the other doped metal elements to manganese element in S1 is 1:6-120, and a molar ratio of the other doped metal elements to iron element is 1:4~80.

6. The method according to claim 1, wherein the precipitant in S2 is an oxalate, and the oxalate comprises one or more of oxalic acid, potassium oxalate, and sodium oxalate.

7. The method according to claim 1, wherein a molar ratio of the mixed metal solution to the precipitant in S2 is 1:1-1.5, the concentration of the precipitant solution is 0.3- 3.0 mol/L, and a bubbling gas used in S3 is one of argon, nitrogen and helium.

8. The method according to claim 1, wherein the complexant is an oxalate, and the oxalate comprises at least one or more of ammonium oxalate, ammonium sulfate and ammonium carbonate.

9. The method according to claim 8, wherein the ammonium oxalate is ammonium oxalate monohydrate.

10. The method according to claim 1, wherein in S4, the heating temperature is 20-100 °C, and a dropwise flow rate of the precipitant is 0.01~1.0 L/min.

11. The method according to claim 1, wherein the inert gas in S5 is one of argon, nitrogen, helium, with a reaction temperature is 20-100 °C and a reaction time is 0.5~48 h.

12. An in situ metal-doped manganese iron oxalate precursor, prepared by the method of any one of claims 1-11.

13. The in situ metal-doped manganese iron oxalate precursor according to claim 12, having a chemical formula MnₓFe_{y}M_{1-x-y}C₂O₄·2H₂O, wherein 0.6≤x≤0.8, 0.3≤y≤0.5, and 0<1-x-y<_0.02; M represents a doped metal element, and the doped metal element is selected from Mg, Zn, Co, Ni, Al, Cu, Zr, Cr, and V.

14. The in situ metal-doped manganese iron oxalate precursor according to claim 12, wherein the chemical formula is Mn_{0.6}Fe_{0.38}Mg_{0.02}C₂O₄·2H₂O.
